# EUROPÄISCHE PATENTSCHRIFT

(11) **EP 1 488 756 B1**
(45) Veröffentlichungstag und Bekanntmachung des Hinweises auf die Patenterteilung: **17.06.2009**
(21) Anmeldenummer: 04008405.5
(22) Anmeldetag: 07.04.2004
(51) Int. Cl.: A61B 18/14

(54) **chirurgisches Koagulationsinstrument**
Surgical coagulation instrument
instrument chirurgical de coagulation

(30) Priorität: 20.06.2003 DE 10328512
(43) Veröffentlichungstag der Anmeldung: 22.12.2004
(73) Patentinhaber: Aesculap AG, 78532 Tuttlingen (DE)
(72) Erfinder: Kupferschmid, Bernhard, Dipl.-Ing., 78576 Emmingen-Liptingen (DE); Mayenberger, Rupert, Dipl.-Ing., 78239 Rielasingen (DE); Weisshaupt, Dieter, Dipl.-Ing., 78194 Immendingen (DE)
(74) Vertreter: Hoeger, Stellrecht & Partner Patentanwälte

(56) Entgegenhaltungen:
- EP-A- 1 153 578
- WO-A-96/22740
- DE-A- 19 855 812

## Beschreibung

Die vorliegende Erfindung betrifft ein chirurgisches Instrument mit mindestens einem Werkzeug umfassend einen Träger und mindestens ein mit dem Träger verklebtes Funktionselement.

Chirurgische Instrumente der eingangs beschriebenen Art sind beispielsweise in Form von Scheren bekannt, die ein metallisches oder aus Kunststoff hergestelltes Scherenblatt umfassen, auf welches eine Schneide aus Keramik oder Metall aufgeklebt ist. Ein solcher zweiteiliger Aufbau des Werkzeugs wird beispielsweise bei Bipolarinstrumenten verwendet, da solche Instrumente elektrisch voneinander isolierte Werkzeuge erfordern. Wird beim Verwenden des Instruments ein Strom über das Werkzeug geleitet, so kann es zu einem Verdampfen des Klebstoffs entlang der Schneidkante kommen.

Aus der WO 96/22740 A1 sowie aus der DE 198 55 812 A1 sind chirurgische endoskopische Bipolarscheren der eingangs beschriebenen Art bekannt.

Es ist daher Aufgabe der vorliegenden Erfindung, ein chirurgisches Instrument der Eingangs beschriebenen Art derart zu verbessern, daß der Träger mit dem Funktionselement einfach und sicher verklebt werden kann unter Aufrechterhaltung der gewünschten Funktion des Instruments.

Diese Aufgabe wird bei einem chirurgischen Instrument der eingangs beschriebenen Art erfindungsgemäß dadurch gelöst, daß der Träger eine erste Anlagefläche aufweist, daß das Funktionselement eine zweite Anlagefläche aufweist und daß die erste Anlagefläche spaltfrei, direkt und klebstoffrei an der zweiten Anlagefläche anliegt.

Eine derartige Ausgestaltung hat den Vorteil, daß bei entsprechender Formgebung des Trägers und des Funktionselements eine definierte Koagulationsstrecke entlang des Übergangsbereichs zwischen dem Träger und dem Funktionselement, beispielsweise einem Metall und einer Keramik, ausgebildet wird, wenn das Instrument als bipolares Instrument eingesetzt wird. Wird ferner ein Strom entlang der Anlageflächen geleitet, so ist kein Verdampfen eines Klebstoffs möglich, da aufgrund des spaltfreien aneinander Anliegens der beiden Anlageflächen beim Verkleben kein Klebstoff zwischen die Anlageflächen in diesem Bereich eindringen kann. Dadurch, dass die erste Anlagefläche direkt und klebstoffrei an der zweiten Anlagefläche anliegt, ist es unmöglich, daß der Klebstoff verdampfen kann, wenn ein Strom über das Werkzeug geleitet wird.

Denkbar wäre es, das Instrument zu unterschiedlichen Zwecke auf vielfache Weise auszugestalten, beispielsweise könnte es als Zange, Fasszange oder Nadelhalter und das mindestens eine Werkzeug entsprechend ausgebildet sein. Vorteilhaft ist es jedoch, wenn das mindestens eine Werkzeug ein eine Schneide umfassendes Scherenblatt ist, wenn die Schneide eine Schneidkante aufweist und wenn die erste und die zweite Anlagefläche im Bereich der Schneidkante direkt aneinander anliegen oder in die Schneidkante übergehen. Durch das spaltfreie aneinander Anliegen des Funktionselements am Träger wird eine genau definierte Schneidkante ausgebildet, welche besonders glatt und ohne unerwünschte Vorsprünge geformt sein kann. Insbesondere kann kein Verbindungsmittel, beispielsweise in Form eines Klebstoffs oder eines anderweitigen Klebemittels, wie zum Beispiel Lötzinn, im Übergangsbereich zwischen dem Träger und dem Funktionselement austreten und so die Form des Übergangsbereich in unerwünschter Weise verändern.

Vorzugsweise liegen die erste Anlagefläche und die zweite Anlagefläche spaltfrei entlang mindestens einer Berührlinie aneinander an. Dadurch wird verhindert, daß beispielsweise zwischen den beiden Anlageflächen zum Verbinden derselben eingebrachter Klebstoff, insbesondere im Bereich der Schneidkante, einen Teil der Oberfläche des Werkzeugs bildet. Dies hätte insbesondere dann den Nachteil, wenn ein Koagulationsstrom entlang dieser Oberfläche des Werkzeugs fließen würde, wodurch der Klebstoff in unerwünschter Weise verdampfen und im menschlichen Körper freigesetzt werden könnte. Zudem könnte dabei auch das Instrument beschädigt werden.

Besonders günstig ist es, wenn die erste Anlagefläche und die zweite Anlagefläche spaltfrei mindestens abschnittsweise im Bereich einer gemeinsamen Berührfläche flächig aneinander anliegen. Insbesondere dann, wenn die beiden Anlageflächen miteinander verklebt werden, wird auf diese Weise eine besonders sichere Klebstoff-freie Trennung zwischen beiden Anlageflächen sichergestellt. Auch bei besonders großen, entlang der Oberfläche des Werkzeugs fließenden Koagulationsströmen wird so ein Verdampfen des Klebstoffs verhindert.

Gemäß einer bevorzugten Ausführungsform der Erfindung kann vorgesehen sein, daß der Träger eine erste Klebefläche aufweist, daß das Funktionselement eine zweite Klebefläche aufweist, daß die erste Klebefläche von der zweiten Klebefläche durch einen Klebespalt getrennt ist und daß der Klebespalt mit einer im wesentlichen von einem Klebstoff gebildeten Klebeschicht gefüllt ist. Diese Ausgestaltung stellt sicher, daß das Funktionselement mit dem Träger in gewünschter Weise verbunden werden kann. Die Ausbildung des Klebespalts ermöglicht ein Einbringen des Klebstoffs zwischen die beiden Klebenflächen des Trägers und des Funktionselements in definierter Weise.

Besonders vorteilhaft ist es, wenn der Klebespalt mindestens abschnittsweise keilförmig ausgebildet ist. Auf diese Weise lassen sich Fertigungstoleranzen von Träger und Funktionselement besonders einfach ausgleichen. Ferner kann dadurch auch eine Anlage der beiden Teile längs einer Berührlinie erreicht werden.

Günstigerweise nimmt eine Dicke des keilförmigen Klebespalts in Richtung auf die aneinander anliegenden ersten und zweiten Anlageflächen ab. Durch eine derartige Ausgestaltung des Klebespalts wird einerseits eine sichere Verbindung zwischen Träger und Funktionselement gewährleistet, andererseits wird eine Klebstoffmenge zwischen den beiden Teilen und benachbart dem spaltfrei aneinander anliegenden Bereich oder der Berührlinie minimiert.

Gemäß einer bevorzugten Ausführungsform der Erfindungen, kann vorgesehen sein, daß die erste Anlagefläche mindestens abschnittsweise eine erste Ebene definiert, daß die zweite Anlagefläche mindestens abschnittsweise eine zweite Ebene definiert und daß die erste Ebene und die zweite Ebene relativ zueinander um einen Neigungswinkel geneigt sind und sich in einer Berührlinie der ersten und zweiten Anlagefläche schneiden. Auf diese Weise läßt sich beispielsweise ein keilförmiger Klebespalt mit den bereits oben beschriebenen Vorteilen ausbilden.

Um das mindestens eine Werkzeug möglichst kompakt zu gestalten, kann es günstig sein, wenn der Neigungswinkel kleiner als 45° ist, vorzugsweise kleiner als 20°. Insbesondere bei sehr kleinen Neigungswinkeln im Bereich kleiner als 20° wird nur eine minimale Menge Klebstoff benötigt, um den Träger und das Funktionselement sicher und dauerhaft miteinander zu verbinden.

Vorzugsweise ist im Klebespalt zwischen der ersten und der zweiten Klebefläche mindestens ein Abstandshalter angeordnet. Der mindestens eine Abstandshalter stellt sicher, daß das Funktionselement relativ zum Träger nicht verkippen kann. Dies könnte passieren, da der Träger und das Funktionselement mit ihren beiden Anlageflächen spaltfrei aneinander anliegen, dagegen im übrigen Bereich der miteinander zu verbindenden Flächen des Trägers und des Funktionselements ein Spalt ausgebildet sein kann. Das mindestens eine Abstandselement definiert somit einen minimalen Abstand zwischen der ersten und der zweiten Klebefläche, so daß die erste Anlagefläche und die zweite Anlagefläche vollflächig aneinander anliegen können.

Besonders einfach wird der Aufbau des Instruments, wenn die erste und/oder die zweite Klebefläche den mindestens einen Abstandshalter tragen. Damit kann zu einem Verkleben Klebstoff entweder in den Klebespalt eingespritzt oder auf eine oder beide Klebeflächen aufgetragen werden, bevor der Träger mit dem Funktionselement in Verbindung gebracht wird. Automatisch wird durch den mindestens einen Abstandshalter ein gewünschter Abstand zwischen dem Funktionselement und dem Träger aufrechterhalten.

Alternativ oder zusätzlich wäre es denkbar, daß der Klebstoff den mindestens einen Abstandshalter enthält. Eine solche Ausgestaltung hat den Vorteil, daß sich sowohl der Träger als auch das Funktionselement auf besonders einfache Weise herstellen lassen und entsprechend von tatsächlich ermittelten Fertigungstoleranzen nach der Herstellung der beiden Teile Abstandshalter mit einer erforderlichen Größe ausgewählt und dem Klebstoff beigemengt werden können oder daß auch direkt ein Klebstoff mit entsprechenden Abstandshaltern ausgewählt wird.

Günstig ist es, wenn eine Vielzahl von Abstandshaltern vorgesehen ist. Auf diese Weise ergeben sich eine Vielzahl von Kontaktpunkten zwischen dem Funktionselement, dem Abstandshalter und dem Träger, so daß der Klebespalt in gewünschter Weise ausgebildet wird. Denkbar sind grundsätzlich eine Vielzahl von Formen für den mindestens einen Abstandshalter. Besonders vorteilhaft ist es, wenn der mindestens eine Abstandshalter kugelförmig, pyramidenförmig, kegelförmig, zylindrisch oder quaderförmig ist. Derartige Formen lassen sich besonders einfach und definiert herstellen.

Grundsätzlich könnten die Klebeflächen des Trägers und des Funktionselements vollständig glatt ausgebildet sein. Gemäß einer bevorzugten Ausführungsform der Erfindung ist jedoch vorgesehen, daß die Oberfläche mindestens einer Klebefläche mindestens einen strukturierten Oberflächenbereich aufweist. Durch Ausbildung einer Struktur auf den Klebeflächen ergibt sich eine Vergrößerung derselben, was zu einer Erhöhung der Klebefestigkeit zwischen dem Träger und dem Funktionselement beiträgt.

Günstig ist es, wenn der strukturierte Oberflächenbereich im wesentliche eine symmetrische Struktur aufweist. Eine solche Ausgestaltung ist besonders einfach herzustellen. Beispielsweise könnten Pyramiden in unterschiedlichen Formen oder Verzahnungen gewählt werden. Der strukturierte Oberflächenbereich kann gleichzeitig als Abstandshalter dienen.

Alternativ oder zusätzlich wäre es auch denkbar, daß der strukturierte Oberflächenbereich mikrostrukturiert ist. Dies könnte beispielsweise mittels Glasperlmattieren realisiert werden. Eine mikrostrukturierte Oberfläche hat zudem den Vorteil, daß Klebstoffmoleküle besonders gut mit dem Träger und/oder dem Funktionselement verbunden werden können.

Zur Erhöhung der Klebefestigkeit ist es von Vorteil, wenn die Oberfläche der ersten und/oder der zweiten Klebefläche größer ist als eine Projektion der ersten und/oder der zweiten Klebefläche auf die jeweils andere Klebefläche. Durch eine derartige Ausgestaltung wird sichergestellt, daß die Klebefläche insgesamt vergrößert wird, was eine Angriffsfläche für den Klebstoff vergrößert.

Grundsätzlich wäre es denkbar, identische Materialien miteinander zu verkleben. Zum Einsatz des Instruments bei elektrochirurgischen Eingriffen ist es von Vorteil, wenn der Träger elektrisch leitend und wenn das Funktionselement elektrisch nichtleitend ist. Das Funktionselement dient in diesem Fall als Isolator. Denkbar wäre auch ein umgekehrter Aufbau des Werkzeugs.

Zur Ausbildung einer Schere ist es günstig, wenn das erste Werkzeug relativ zu einem zweiten Werkzeug beweglich, insbesondere verschwenkbar gelagert ist.

Damit das Instrument auch bei endoskopischen chirurgischen Eingriffen verwendet werden kann, kann gemäß einer bevorzugten Ausführungsform der Erfindung vorgesehen sein, daß das Instrument ein Rohrschaftinstrument mit einem langgestreckten Schaft ist, und daß das mindestes eine Werkzeug am distalen Ende des Schafts beweglich gelagert ist.

Günstig ist es, wenn das Instrument eine Schere ist. Dies ermöglicht es, das Instrument beispielsweise als Bipolarschere einzusetzen.

Die nachfolgende Beschreibung bevorzugter Ausführungsformen der Erfindung dient im Zusammenhang mit der Zeichnung der näheren Erläuterung. Es zeigen:
- Figur 1:: eine Längsschnittansicht einer erfindungsgemäßen Schere;
- Figur 2:: eine vergrößerte Ansicht des Ausschnitts A in Figur 1;
- Figur 3:: eine gegenüber der in Figur 2 um 90° gedrehte Längsschnittansicht einer Schere mit geschlossenen Scherenblättern;
- Figur 4:: eine vergrößerte Ansicht eines Scherenblatts;
- Figur 5:: eine Schnittansicht längs Linie 5-5 in Figur 4;
- Figur 6:: eine Schnittansicht längs Linie 6-6 in Figur 5;
- Figur 7:: eine zweites Ausführungsbeispiel eines erfindungsgemäßen Scherenblatts in einer Ansicht ähnlich Figur 6;
- Figur 8:: ein drittes Ausführungsbeispiel eines Scherenblatts in einer Ansicht ähnlich Figur 6;
- Figur 9:: ein viertes Ausführungsbeispiel eines Scherenblatts in einer Ansicht ähnlich Figur 5; und
- Figur 10:: ein fünftes Ausführungsbeispiel eines Scherenblatts in einer Ansicht ähnlich Figur 5.

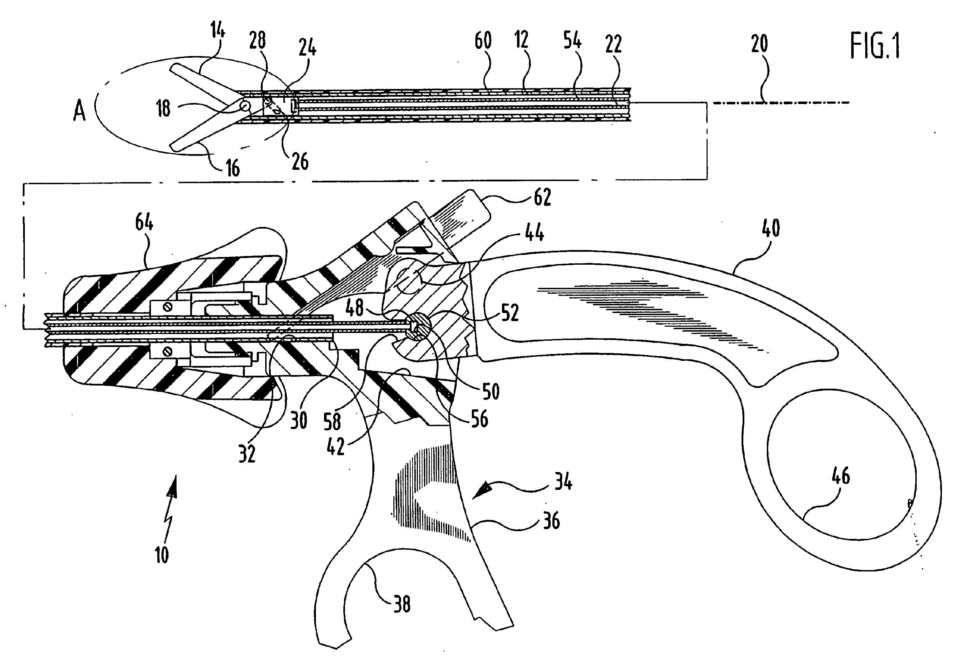

In Figur 1 ist eine Längsschnittansicht einer insgesamt mit dem Bezugszeichen 10 versehenen, als endoskopisches Rohrschaftinstrument ausgebildeten Bipolarschere dargestellt.

Die Bipolarschere 10 umfaßt einen langgestreckten, rohrförmigen Schaft 12, an dessen distalem Ende zwei relativ zueinander verschwenkbare Scherenblätter 14 und 16 auf einem Lagerpin 18 gelagert sind, welcher den Schaft 12 beidseitig und quer zu einer Längsachse 20 des Schafts 12 durchsetzt.

Zum Bewegen der Scherenblätter 14 und 16 ist an einem distalen Ende einer in Richtung der Längsachse 20 im Schaft 12 längsverschieblichen Schub- und Zugstange 22 ein Antriebskörper 24 angeordnet, welcher mit zwei Führungsschlitzen 26 versehen ist, in die quer zur Längsachse 20 von den Scherenblättern 14 und 16 abstehende Lagerzapfen 28 eintauchen und in Folge einer axialen Verschiebung des Antriebskörpers 24 zwangsgeführt werden, wodurch die Scherenblätter 14 und 16 geöffnet beziehungsweise geschlossen werden.

An seinem proximalen Ende 30 ist der Schaft 12 in einer Längsbohrung 32 eines feststehenden Griffteils 34 aufgenommen, von dem sich eine feststehende Branche 36 mit einer Fingeröffnung 38 im wesentlichen quer zur Längsachse 20 von dieser weg erstreckt. Am Griffteil 34 ist eine zweite Branche 40 in einer in proximaler Richtung offenen Ausnehmung 42 um einen quer zur Längsachse 20 die Ausnehmung 42 durchsetzenden Lagerbolzen 44 schwenkbar gelagert und weist an ihrem freie Ende eine weitere Fingeröffnung 46 auf.

Ein proximales Ende der Schub- und Zugstange 22 ist mit einem kurzen zylindrischen Kopf 48 versehen, welcher in einer sich einstufig erweiternden Lagernut 50 eines Lagerzylinders 52 formschlüssig eingreift und darin gehalten ist.

Aus der Lagernut 50 ragt die Schub- und Zugstange 22 hervor, die mit einer elektrischen Isolierschicht 54 bedeckt ist. Eine Längsachse des Lagerzylinders 52 verläuft quer zur Längsachse 20. Der Lagerzylinder 52 wird an der Branche 40 in der Nähe des Lagerbolzens 44 in einer Lagerbohrung 56 gehalten, welche sich quer zur Längsachse 20 erstreckt und einen sich vom Zentrum der Lagerbohrung 56 in distaler Richtung erweiternden Schlitz 58 umfaßt. Ebenso wie die Schub- und Zugstange 22 ist auch der Schaft 12 von einer elektrisch isolierenden Schicht 60 umgeben. Sowohl der Schaft 12 als auch die Schub- und Zugstange 22 sind in nicht näher dargestellter Weise mit einem bipolaren Anschluß 62 verbunden, über welchen die Bipolarschere 10 mittels Leitungen an eine elektrische Energieversorgungseinheit angeschlossen werden kann. Sowohl über die Schub- und Zugstange 22 als auch über den Schaft 12 wird jeweils eine elektrische Verbindung zu einem der beiden Scherenblättern 14 und 16 hergestellt, die relativ zueinander isoliert sind. Dies ermöglicht es, zum Koagulieren von Gewebe beispielsweise einen Hochfrequenzstrom über die Scherenblätter 14 und 16 zu leiten und das koagulierte Gewebe im Anschluß an den Koagulationsvorgang zu durchtrennen.

Des weiteren ist ein relativ zum Schaft 12 drehfester Drehknopf 64 vorgesehen, welcher jedoch relativ zum Griffteil verdrehbar ist, so daß das distale Ende der Bipolarschere 10 mit den beiden Scherenblättern 14 und 16 relativ zu den beiden Branchen 36 und 40 um die Längsachse 20 verdreht werden kann.

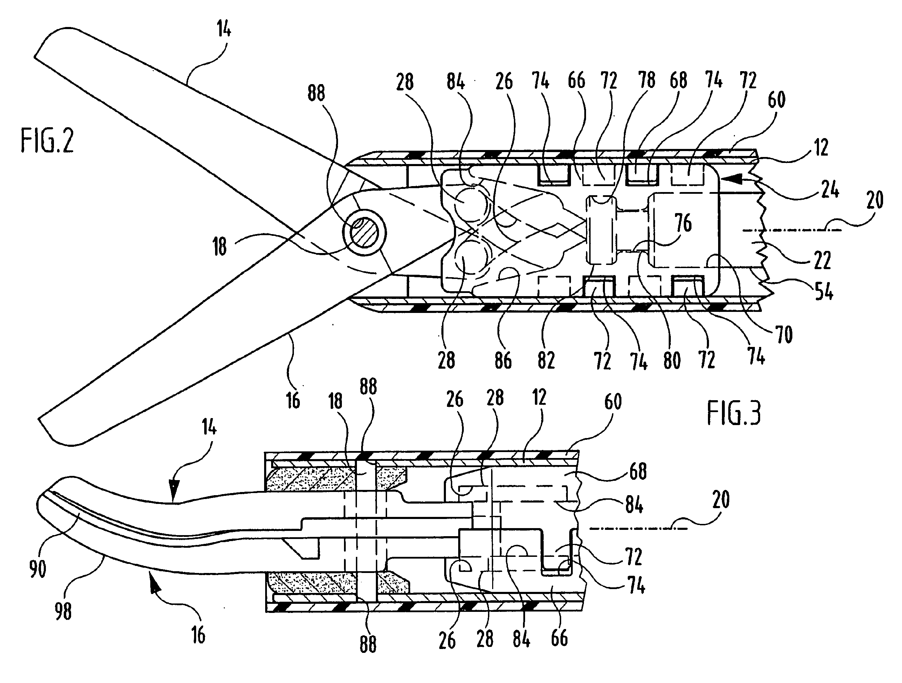

Figur 2 zeigt eine Vergrößerung des Ausschnitts A in Figur 1. Zum Bewegen der beiden Scherenblätter 14 und 16 dient der Antriebskörper 24, welcher aus zwei Halbschalen 66 und 68 zusammengesetzt ist. Die beiden Halbschalen 66 und 68 sind identisch aufgebaut und im wesentlichen in Form eines halben, in Längsrichtung durchtrennten Zylinders ausgebildet. Zum Zusammensetzen weisen beide Halbschalen 66 und 68 jeweils zwei Paare in Umfangsrichtung vorstehender Verbindungszapfen 72 auf, sowie zwei Paare diese aufnehmende Aussparungen 74. Die Aussparungen 74 und die Verbindungszapfen 72 sind abwechselnd entlang des parallel zur Längsachse 20 verlaufenden Randes der Halbschalen 66 und 68 ausgebildet. Im zusammengesetzten Zustand, also in einer Kupplungsstellung, in welcher die beiden Halbschalen 66 und 68 miteinander verbunden sind und die Schub- und Zugstange 22 mit den beiden Scherenblättern 14 und 16 verbinden, bilden die beiden Halbschalen 66 und 68 proximalseitig eine Stangenaufnahme 70 in Form einer Bohrung aus. Diese verjüngt sich einstufig auf einem kurzen Abschnitt und erweitert sich dann wieder im Durchmesser einstufig, so daß ein Ringvorsprung 76 zwischen der Stangenaufnahme 70 und einer Ringnut 78 gebildet wird.

Ein distales Ende der Schub- und Zugstange 22 weist eine zum Ringvorsprung 76 korrespondierende Ringnut 80 und einen sich daran anschließenden zylinderförmigen Kopf 82 auf, welcher korrespondierend zur Ringnut 78 ausgebildet ist.

Distalseitig sind beide Halbschalen 66 und 68 mit im wesentlichen V-förmigen, in distaler Richtung offenen Ausnehmungen 84 und 86 versehen, wobei in jeder Ausnehmung in Richtung auf den Schaft 12 jeweils einer der beiden Führungsschlitze 26 eingearbeitet ist. Die beiden Führungsschlitze 26 bilden eine quer zur Längsachse 20 und auf diese hin offene Nut, in welche die Lagerzapfen 28 der Scherenblätter 14 und 16 jeweils quer zur Längsachse 20 und von dieser weg weisend eintauchen. Die Führungsschlitze 26 sind leicht gekrümmt.

Zum Zusammenbauen des vorderen Endes der Bipolarschere 10 werden zunächst die beiden Scherenblätter 14 und 16 mit ihren Lagerzapfen 28 in die jeweiligen Führungsschlitze 26 eingesetzt und die Schub- und Zugstange 22 mit ihrem Kopf 82 in die Ringnut 78 eingelegt. Die Halbschalen 66 und 68 werden quer zur Längsachse 22 zusammengeschoben, so daß jeder Verbindungszapfen 72 der Halbschalen 66 in eine Aussparung 74 der Halbschale 68 eintaucht und umgekehrt. Die Halbschalen 66 und 68 werden nicht miteinander verklebt oder auf andere Weise unlösbar miteinander verbunden.

Nach dem beschriebenen Zusammenfügen der Teile wird die Schub- und Zugstange 22 von distal her kommend in den Schaft 12 eingeführt, bis der Schaft 12 die Halbschalen 66 und 68 umgibt. Sobald die Halbschalen 66 und 68 in den Schaft 12 eingeführt sind, sind die Schub- und Zugstange 22 und die Scherenblättern 14 in axialer Richtung unlösbar miteinander verbunden, denn der Schaft 12 sichert die Halbschalen 66 und 68 gegen ein Lösen von der Schub- und Zugstange 22. Abschließend wird der Lagerpin 18 durch quer zur Längsachse verlaufende Bohrungen 88 im Schaft 12 hindurchgesteckt, wodurch die Scherenblätter 14 und 16 am Schaft 12 festgelegt werden, wodurch aufgrund einer Bewegung der Schub- und Zugstange in axialer Richtung und durch Führung der Lagerzapfen 28 in den Führungsschlitzen 26 nur noch eine Schwenkbewegung der Scherenblätter 14 und 16 aufeinander zu beziehungsweise voneinander weg möglich ist.

In Figur 3 ist zu erkennen, daß die beiden Scherenblätter 14 und 16 leicht gekrümmt sind. Ferner ist dargestellt, daß das Scherenblatt 16 mit seiner in Richtung auf das Scherenblatt 14 hin weisenden Seite mit einer elektrisch isolierenden, ein Funktionselement bildenden Keramikschicht 90 versehen ist. In ähnlicher Weise ist das Scherenblatt 14 mit einer Keramikschicht 92 versehen, welcher in Richtung auf das Scherenblatt 16 hin weist.

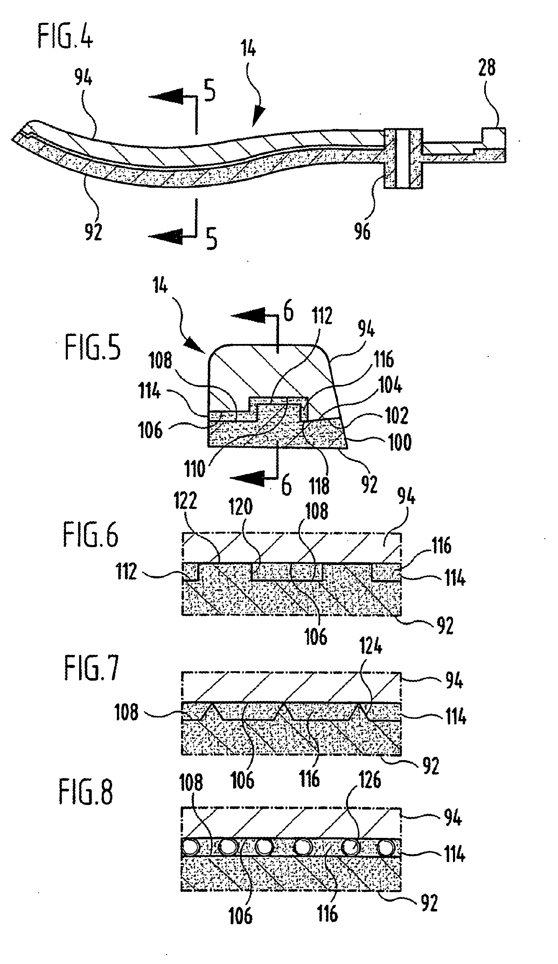

In den Figuren 4 bis 8 ist jeweils dargestellt, daß das Scherenblatt 14 in Form eines metallischen Trägers 94 mit der Keramikschicht 92 verklebt ist. Zur Isolierung der beiden Scherenblätter 14 und 16 voneinander ist die Keramikschicht 92 im Bereich des Lagerpins 18 als Lagerbüchse 96 ausgebildet, so daß es zu keinem Kurzschluß zwischen dem Träger 94 des Scherenblatts und einem metallischen Träger 98 des Scherenblatts 16 kommen kann.

Figur 5 zeigt den besonderen Aufbau des Scherenblatts 14 im Querschnitt. Der Träger 94 und die Keramikschicht 92 liegen nur im Bereich einer Schneidkante 100 direkt und somit spaltfrei aneinander an. Zwischen aneinander anliegenden Kontaktflächen 102 des Trägers und 104 der Keramikschicht besteht also kein Spalt und somit kann auch kein Klebstoff zwischen diese beiden Schichten eindringen.

Die weitere, auf die Keramikschicht 92 hin weisende, nicht als Kontaktfläche 102 dienende Oberfläche des Trägers 94 bildet eine Klebefläche 106, die weitere Oberfläche der Keramikschicht 92 eine Klebefläche 108. Die Klebefläche 106 des Trägers 94 weist eine flache Klebenut 110 auf, in welche ein Klebevorsprung 112 der Keramikschicht 92 eintaucht, diese jedoch nicht formschlüssig ausfüllt. Auf diese Weise wird ein Klebespalt 114 zwischen dem Träger 94 und der Keramikschicht 92 gebildet, welcher zudem den Klebevorsprung 112 umgibt.

Zum Verbinden des Trägers 94 mit der Keramikschicht 92 wird ein geeigneter Klebstoff in den Klebespalt 114 eingebracht.

Um ein Verkippen der Keramikschicht 92 relativ zum Träger 94 um eine Kante 118 des Träger 94, welche die Kontaktfläche 102 zum Klebespalt 114 hin begrenzt, zu verhindern, sind auf dem Klebevorsprung 112 oder auf einer der Klebeflächen 106 oder 108 zusätzliche Abstandshalter 120 in regelmäßigen Abständen in Form von länglichen kleinen Quadern angeordnet, deren Höhe der Breite des Klebespalts 114 entspricht. Stirnflächen 122 der Abstandshalter 120 liegen dann spaltfrei direkt an der Klebefläche 106 des Trägers 94 an.

Alternative Ausgestaltungen von Abstandshaltern sind in den Figuren 7 und 8 dargestellt. In Figur 7 sind Abstandshalter 124 in Form kleiner Pyramiden einstückig an die Keramik angeformt, wobei Spitzen der Abstandshalter 124 die Klebefläche 106 des Trägers 94 punktförmig berühren. Auf diese Weise kann bei Einhaltung des gewünschten Abstands zwischen den beiden Klebeflächen 106 und 108 die mit Klebstoff 116 bedeckte Klebefläche 106 des Trägers 94 maximiert werden.

Eine dritte Variante für einen möglichen Abstandshalter ist in Figur 8 dargestellt. Zur Einhaltung des Abstands zwischen der Klebefläche 106 des Trägers 94 und der Klebefläche 108 der Keramik 92 sind eine Vielzahl von Kugeln 126 mit dem Klebstoff 116 vermischt, wobei der Durchmesser der Kugel 116 so gewählt ist, daß er der gewünschten Breite des Klebespalts 114 entspricht. Aufgrund ihrer Form verteilen sich die Kugeln 126 mehr oder weniger gleichmäßig zwischen den Klebeflächen 106 und 108.

Des weiteren kann zusätzlich die Klebefläche 106 und ebenso die Klebefläche 108 in beliebiger Weise strukturiert oder mikrostrukturiert sein, um die mit Klebstoff 116 benetzte und bedeckte Fläche zu vergrößern. Dies ist im Querschnitt in Figur 5 beispielhaft durch Ausbildung der Klebenut 110 und des Klebevorsprungs 112 angedeutet.

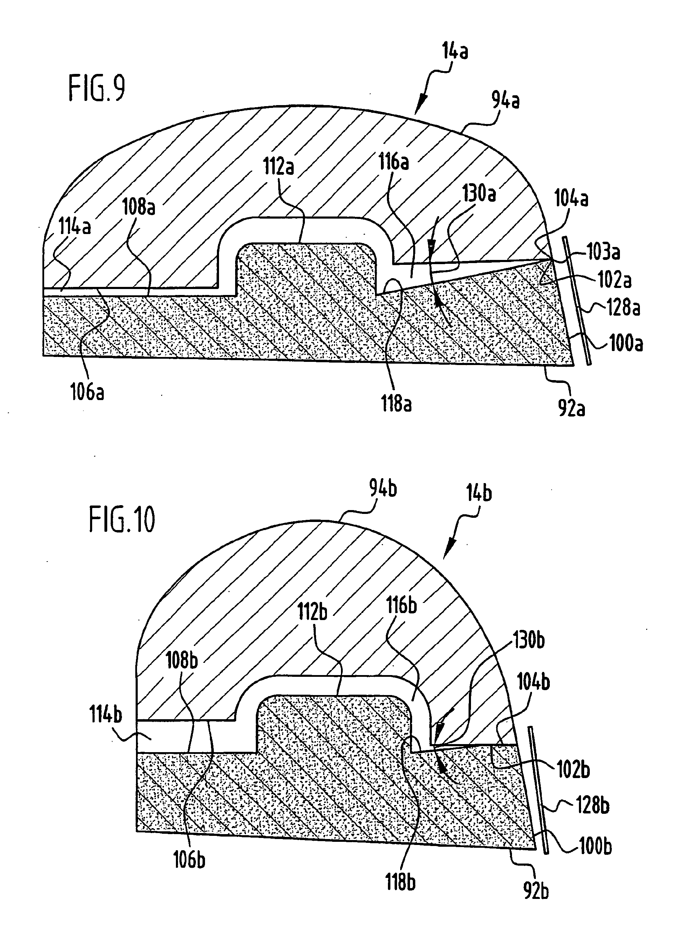

In Figur 9 ist eine Querschnittsansicht einer vierten Variante eines Scherenblatts dargestellt, wie es im Zusammenhang mit den Figuren 1 bis 5 beschrieben ist. Daher werden identische oder im Aufbau sehr ähnliche Elemente des Scherenblatts mit identischen Bezugszeichen unter Hinzufügung des Suffix "a" bezeichnet.

Wesentlicher Unterschied zu dem im Zusammenhang mit Figur 5 beschriebenen Ausführungsbeispiel ist, daß die beiden Kontaktflächen 102a und 104a strenggenommen keine Kontaktflächen bilden, sondern Kontaktlinien. Auf diese Weise wird insgesamt eine gemeinsame Kontaktlinie 103a ausgebildet, die den Klebespalt 114a, der sich in Richtung auf die Kontaktlinie 103a keilförmig zuspitzt, im Bereich der Schneidkante 100a schließt. Dadurch wird ein entlang der Schneidkante 100a längs einer Koagulationsstrecke 128a fließender Strom bei Verwendung eines mit dem beschriebenen Scherenblatt ausgebildeten Instruments nicht in Kontakt mit dem zwischen den Klebeflächen 106a und 108a in den Klebespalt 114a eingebrachten Klebstoff 116a gelangen und zu einer Verdampfung desselben führen. Die beiden Kontaktflächen 104a und 102a sind relativ zueinander um einen Neigungswinkel 130a geneigt, der in etwa einen Wert von 10° aufweist.

Ein fünftes Ausführungsbeispiel für ein erfindungsgemäßes Scherenblatt ist in Figur 10 im Querschnitt dargestellt. Wie schon bei dem in Figur 9 dargestellten Ausführungsbeispiel sind identische oder ähnliche Elemente zu denen des im Zusammenhang mit den Figuren 1 bis 5 beschriebenen Ausführungsbeispiels mit identischen Bezugszeichen unter Hinzufügung des Suffix "b" bezeichnet.

Das fünfte Ausführungsbeispiel stimmt in seinem grundlegenden Aufbau mit dem vierten Ausführungsbeispiel überein, unterscheidet sich jedoch dadurch, daß die Kontaktflächen 102b und 104b in Form von jeweils zwei ebenen Flächenbereichen ausgebildet sind, so daß sich eine flächige Berührung der beiden Kontaktflächen 102b und 104b ausgehend von der Schneidkante 100b ergibt. Die Kontaktfläche 102b ist dabei vollständig eben ausgebildet, die Kontaktfläche 104b weist zwei relativ zueinander geneigte Flächenabschnitte auf, wobei der eine Flächenabschnitt parallel zur Kontaktfläche 102b verläuft und an dieser anliegt, der andere Kontaktflächenbereich relativ zur Kontaktfläche 102b um einen Neigungswinkel 130b geneigt ist, so daß sich auch hier ein keilförmiger Klebespalt zwischen den geneigten Flächenabschnitten der Kontaktflächen 102b und 104b ausbilden kann. Der Neigungswinkel 130b weist beim vorliegenden Ausführungsbeispiel einen Wert von etwa 10° auf. Eine Koagulationsstrecke 128b entlang der Schneidkante 100b ist durch die flächige Berührung der aneinander anliegenden Kontaktflächen 102b und 104b noch weiter von dem den Träger 94b mit der Keramikschicht 92b verbindenden Klebstoff 116b entfernt, so daß ein Verdampfen desselben praktisch unmöglich ist.

Die beiden Ausführungsvarianten, wie sie in den Figuren 9 und 10 beschrieben sind, können ferner mit Abstandshaltern versehen sein, wie sie im Zusammenhang mit den Figuren 6 bis 8 näher erläutert sind.

## Patentansprüche

1. Chirurgisches Instrument (10) mit mindestens einem Werkzeug (14, 16) umfassend einen Träger (94, 98) und mindestens ein mit dem Träger (94, 98) verklebtes Funktionselement (90, 92), welcher Träger (94, 98) eine erste Anlagefläche (102) aufweist, welches Funktionselement (90, 92) eine zweite Anlagefläche (104) aufweist **dadurch gekennzeichnet, daß** die erste Anlagefläche (102) spaltfrei, direkt und klebstofffrei an der zweiten Anlagefläche (104) anliegt.

2. Instrument nach einem der voranstehenden Ansprüche, **dadurch gekennzeichnet, daß** das mindestens eine Werkzeug (14, 16) ein eine Schneide umfassendes Scherenblatt ist, daß die Schneide eine Schneidkante (100) aufweist und daß die erste und die zweite Anlagefläche (102, 104) im Bereich der Schneidkante (100) direkt aneinander anliegen oder in die Schneidkante (100) übergehen.

3. Instrument nach einem der Ansprüche 1 oder 2, **dadurch gekennzeichnet, daß** die erste Anlagefläche und die zweite Anlagefläche spaltfrei entlang mindestens einer Berührlinie aneinander anliegen.

4. Instrument nach einem der voranstehenden Ansprüche, **dadurch gekennzeichnet, daß** die erste Anlagefläche und die zweite Anlagefläche spaltfrei mindestens abschnittsweise im Bereich einer gemeinsamen Berührfläche flächig aneinander anliegen.

5. Instrument nach einem der voranstehenden Ansprüche, **dadurch gekennzeichnet, daß** der Träger (94, 98) eine erste Klebefläche (106) aufweist, daß das Funktionselement (90, 92) eine zweite Klebefläche (108) aufweist, daß die erste Klebefläche (106) von der zweiten Klebefläche (108) durch einen Klebespalt (114) getrennt ist und daß der Klebespalt (114) mit einer im wesentlichen von einem Klebstoff (116) gebildeten Klebeschicht gefüllt ist.

6. Vorrichtung nach Anspruch 5, **dadurch gekennzeichnet, daß** der Klebespalt mindestens abschnittsweise keilförmig ausgebildet ist.

7. Vorrichtung nach Anspruch 6, **dadurch gekennzeichnet, daß** eine Dicke des keilförmigen Klebespalts in Richtung auf die aneinander anliegenden ersten und zweiten Anlageflächen abnimmt.

8. Vorrichtung nach einem der voranstehenden Ansprüche, **dadurch gekennzeichnet, daß** die erste Anlagefläche mindestens abschnittsweise eine erste Ebene definiert, daß die zweite Anlagefläche mindestens abschnittsweise eine zweite Ebene definiert und daß die erste Ebene und die zweite Ebene relativ zueinander um einen Neigungswinkel geneigt sind und sich in einer Berührlinie der ersten und zweiten Anlageflächen schneiden.

9. Vorrichtung nach Anspruch 8, **dadurch gekennzeichnet, daß** der Neigungswinkel kleiner als 45° ist, vorzugsweise kleiner als 20°.

10. Instrument nach einem der Ansprüche 5 bis 9, **dadurch gekennzeichnet, daß** im Klebespalt (114) zwischen der ersten und der zweiten Klebefläche (106, 108) mindestens ein Abstandshalter (120; 124; 126) angeordnet ist.

11. Instrument nach Anspruch 10, **dadurch gekennzeichnet, daß** die erste und/oder die zweite Klebefläche (106, 108) den mindestens einen Abstandshalter (120; 124) tragen.

12. Instrument nach einem der Ansprüche 10 oder 11, **dadurch gekennzeichnet, daß** der Klebstoff (116) den mindestens einen Abstandshalter (126) enthält.

13. Instrument nach einem der Ansprüche 10 bis 12, **dadurch gekennzeichnet, daß** eine Vielzahl von Abstandshaltern (120; 124; 126) vorgesehen ist.

14. Instrument nach einem der Ansprüche 10 bis 13, **dadurch gekennzeichnet, daß** der mindestens eine Abstandshalter (120; 124; 126) kugelförmig, pyramidenförmig, kegelförmig, zylindrisch oder quaderförmig ist.

15. Instrument nach einem der Ansprüche 5 bis 14, **dadurch gekennzeichnet, daß** die Oberfläche mindestens einer Klebefläche (106, 108) mindestens einen strukturierten Oberflächenbereich (110; 112) aufweist.

16. Instrument nach Anspruch 15, **dadurch gekennzeichnet, daß** der strukturierte Oberflächenbereich (110; 112) im wesentlichen eine symmetrische Struktur aufweist.

17. Instrument nach einem der Ansprüche 15 oder 16, **dadurch gekennzeichnet, daß** der strukturierte Oberflächenbereich mikrostrukturiert ist.

18. Instrument nach einem der Ansprüche 5 bis 17, **dadurch gekennzeichnet, daß** eine Oberfläche der ersten und/oder der zweiten Klebefläche (106, 108) größer ist als eine Projektion der ersten und/oder der zweiten Klebefläche (106, 108) in Richtung auf die jeweils andere Klebefläche (106, 108).

19. Instrument nach einem der voranstehenden Ansprüche, **dadurch gekennzeichnet, daß** der Träger (94, 98) elektrisch leitend und daß das Funktionselement (90, 92) elektrisch nichtleitend ist.

20. Instrument nach Anspruch 19, **dadurch gekennzeichnet, daß** das elektrisch nichtleitende Funktionselement (90, 92) eine Keramik ist und daß der elektrisch leitende Träger (94, 98) aus einem Metall hergestellt ist.

21. Instrument nach einem der voranstehenden Ansprüche, **dadurch gekennzeichnet, daß** ein erstes Werkzeug (14) relativ zu einem zweiten Werkzeug (16) beweglich, insbesondere verschwenkbar gelagert ist.

22. Instrument nach einem der voranstehenden Ansprüche, **dadurch gekennzeichnet, daß** das Instrument (10) ein Rohrschaftinstrument mit einem langgestreckten Schaft (12) ist und daß das mindestens eine Werkzeug (14, 16) am distalen Ende des Schafts (12) beweglich gelagert ist.

23. Instrument nach einem der voranstehenden Ansprüche, **dadurch gekennzeichnet, daß** das Instrument (10) eine Schere ist.

## Claims

1. Surgical instrument (10) with at least one tool (14, 16) comprising a carrier (94, 98) and at least one functional component (90, 92) adhesively bonded to the carrier (94, 98), the carrier (94, 98) comprising a first contact surface (102), and the functional component (90, 92) comprising a second contact surface (104), **characterized in that** the first contact surface (102) bears directly and without adhesive on the second contact surface (104) with no gap therebetween.

2. Instrument in accordance with any one of the preceding claims, **characterized in that** the at least one tool (14, 16) is a scissor blade comprising a cutter, **in that** the cutter comprises a cutting edge (100), and **in that** the first and second contact surfaces (102, 104) bear in the area of the cutting edge (100) directly on each other or pass into the cutting edge (100).

3. Instrument in accordance with Claim 1 or 2, **characterized in that** the first contact surface and the second contact surface bear on each other along at least one contacting line with no gap therebetween.

4. Instrument in accordance with any one of the preceding claims, **characterized in that** the first contact surface and the second contact surface bear surface-to-surface on each other at least section-wise in the area of a common contacting surface with no gap therebetween.

5. Instrument in accordance with any one of the preceding claims, **characterized in that** the carrier (94, 98) has a first adhesive surface (106), **in that** the functional component (90, 92) has a second adhesive surface (108), **in that** the first adhesive surface (106) is separated from the second adhesive surface (108) by an adhesive gap (114), and **in that** the adhesive gap (114) is filled with an adhesive layer which is substantially formed by an adhesive (116).

6. Instrument in accordance with Claim 5, **characterized in that** the adhesive gap is of wedge-shaped construction at least section-wise.

7. Instrument in accordance with Claim 6, **characterized in that** a thickness of the wedge-shaped adhesive gap decreases in the direction towards the first and second contact surfaces bearing on each other.

8. Instrument in accordance with any one of the preceding claims, **characterized in that** the first contact surface defines at least section-wise a first plane, **in that** the second contact surface defines at least section-wise a second plane, and **in that** the first plane and the second plane are inclined at an angle of inclination relative to each other and intersect in a contacting line of the first and second contact surfaces.

9. Instrument in accordance with Claim 8, **characterized in that** the angle of inclination is less than 45°, preferably less than 20°.

10. Instrument in accordance with any one of Claims 5 to 9, **characterized in that** at least one spacer (120; 124; 126) is arranged in the adhesive gap (114) between the first and second adhesive surfaces (106, 108).

11. Instrument in accordance with Claim 10, **characterized in that** the first adhesive surface (106) and/or the second adhesive surface (108) carry the at least one spacer (120; 124).

12. Instrument in accordance with Claim 10 or 11, **characterized in that** the adhesive (116) contains the at least one spacer (126).

13. Instrument in accordance with any one of Claims 10 to 12, **characterized in that** a plurality of spacers (120; 124; 126) are provided.

14. Instrument in accordance with any one of Claims 10 to 13, **characterized in that** the at least one spacer (120; 124; 126) is of spherical, pyramidal, conical, cylindrical or parallelepipedal shape.

15. Instrument in accordance with any one of Claims 5 to 14, **characterized in that** the surface of at least one adhesive surface (106, 108) comprises at least one structured surface portion (110; 112).

16. Instrument in accordance with Claim 15, **characterized in that** the structured surface portion (110; 112) essentially has a symmetrical structure.

17. Instrument in accordance with Claim 15 or 16, **characterized in that** the structured surface portion is microstructured.

18. Instrument in accordance with any one of claims 5 to 17, **characterized in that** a surface of the first adhesive surface (106) and/or the second adhesive surface (108) is larger than a projection of the first adhesive surface (106) and/or the second adhesive surface (108) in the direction towards the respective other adhesive surface (106, 108).

19. Instrument in accordance with any one of the preceding claims, **characterized in that** the carrier (94, 98) is electrically conductive, and **in that** the functional component (90, 92) is electrically non-conductive.

20. Instrument in accordance with Claim 19, **characterized in that** the electrically non-conductive functional component (90, 92) is made of a ceramic material, and **in that** the electrically conductive carrier (94, 98) is made of a metal.

21. Instrument in accordance with any one of the preceding claims, **characterized in that** a first tool (14) is movable relative to a second tool (16), in particular, mounted for pivotal movement relative to a second tool (16).

22. Instrument in accordance with any one of the preceding claims, **characterized in that** the instrument (10) is a tubular shaft instrument with an elongated shaft (12), and **in that** the at least one tool (14, 16) is movably mounted at the distal end of the shaft (12).

23. Instrument in accordance with any one of the preceding claims, **characterized in that** the instrument (10) is scissors.

## Revendications

1. Instrument chirurgical (10) comprenant au moins un outil (14, 16) englobant un support (94, 98) et au moins un élément fonctionnel (90, 92) collé avec le support (94, 98), ledit support (94, 98) présentant une première surface d'appui (102) et ledit élément fonctionnel (90, 92) présentant une deuxième surface d'appui (104), **caractérisé en ce que** la première surface d'appui (102) s'appuie sans interstice, directement et sans colle contre la deuxième surface d'appui (104).

2. Instrument selon l'une des revendications précédentes, **caractérisé en ce que** ledit outil ou lesdits outils (14, 16) est ou sont une lame de ciseaux présentant une lame de coupe, **en ce que** la lame de coupe présente un tranchant de coupe (100), et **en ce que** la première et la deuxième surface d'appui (102, 104) s'appuient directement mutuellement l'une contre l'autre ou se raccordent directement au tranchant de coupe (100).

3. Instrument selon l'une des revendications 1 ou 2, **caractérisé en ce que** la première surface d'appui et la deuxième surface d'appui s'appuient l'une contre l'autre sans interstice, le long d'au moins une ligne de contact.

4. Instrument selon l'une des revendications précédentes, **caractérisé en ce que** la première surface d'appui et la deuxième surface d'appui s'appuient l'une contre l'autre sans interstice, au moins par secteurs dans la zone d'une surface de contact commune.

5. Instrument selon l'une des revendications précédentes, **caractérisé en ce que** le support (94, 98) présente une première surface de collage (106), **en ce que** l'élément fonctionnel (90, 92) présente une deuxième surface de collage (108), **en ce que** la première surface de collage (106) est séparée de la deuxième surface de collage (108) par un interstice de collage (114), et **en ce que** l'interstice de collage (114) est rempli d'une couche de collage formée essentiellement par une colle (116).

6. Dispositif selon la revendication 5, **caractérisé en ce que** l'interstice de collage est réalisé en forme de coin au moins par secteurs.

7. Dispositif selon la revendication 6, **caractérisé en ce qu'**une épaisseur de l'interstice de collage en forme de coin, diminue en direction des première et deuxième surfaces d'appui en appui mutuel.

8. Dispositif selon l'une des revendications précédentes, **caractérisé en ce que** la première surface d'appui définit au moins par secteurs un premier plan, **en ce que** la deuxième surface d'appui définit au moins par secteurs un deuxième plan, et **en ce que** le premier plan et le deuxième plan sont inclinés relativement l'un par rapport à l'autre d'un angle d'inclinaison et se coupent le long d'une ligne de contact de la première et de la deuxième surface d'appui.

9. Dispositif selon la revendication 8, **caractérisé en ce que** l'angle d'inclinaison est inférieur à 45°, de préférence inférieur à 20°.

10. Instrument selon l'une des revendications 5 à 9, **caractérisé en ce que** dans l'interstice de collage (114) entre la première et la deuxième surface de collage (106, 108) est agencée au moins une entretoise d'espacement (120 ; 124 ; 126).

11. Dispositif selon la revendication 10, **caractérisé en ce que** la première et/ou la deuxième surface de collage (106, 108) portent ladite ou lesdites entretoises d'espacement (120 ; 124).

12. Instrument selon l'une des revendications 10 ou 11, **caractérisé en ce que** la colle (116) renferme ladite ou lesdites entretoises d'espacement (126).

13. Instrument selon l'une des revendications 10 à 12, **caractérisé en ce que** sont prévues un grand nombre d'entretoises d'espacement (120 ; 124 ; 126).

14. Instrument selon l'une des revendications 10 à 13, **caractérisé en ce que** ladite ou lesdites entretoises d'espacement (120 ; 124 ; 126) sont de forme sphérique, de forme pyramidale, de forme conique, de forme cylindrique ou de forme parallélépipédique.

15. Instrument selon l'une des revendications 5 à 14, **caractérisé en ce que** la superficie d'au moins une surface de collage (106, 108) présente au moins une zone de surface structurée (110 ; 112).

16. Instrument selon la revendication 15, **caractérisé en ce que** la zone de surface structurée (110 ; 112) présente sensiblement une structure symétrique.

17. Instrument selon l'une des revendications 15 ou 16, **caractérisé en ce que** la zone de surface structurée est microstructurée.

18. Instrument selon l'une des revendications 5 à 17, **caractérisé en ce qu'**une superficie de la première et/ou de la deuxième surface de collage (106, 108) est plus grande qu'une projection de la première et/ou de la deuxième surface de collage (106, 108) en direction respectivement de l'autre surface de collage (106, 108).

19. Instrument selon l'une des revendications précédentes, **caractérisé en ce que** le support (94, 98) est électriquement conducteur et **en ce que** l'élément fonctionnel (90, 92) n'est pas électriquement conducteur.

20. Instrument selon la revendication 19, **caractérisé en ce que** l'élément fonctionnel (90, 92) électriquement non conducteur est une céramique, et **en ce que** le support (94, 98) électriquement conducteur est fabriqué en un métal.

21. Instrument selon l'une des revendications précédentes, **caractérisé en ce qu'**un premier outil (14) est monté mobile, notamment pivotant, par rapport à un deuxième outil (16).

22. Instrument selon l'une des revendications précédentes, **caractérisé en ce que** l'instrument (10) est un instrument à corps tubulaire comprenant un corps allongé (12), et **en ce que** ledit ou lesdits outils (14, 16) est ou sont montés à l'extrémité distale du corps (12).

23. Instrument selon l'une des revendications précédentes, **caractérisé en ce que** l'instrument (10) est une paire de ciseaux.
